# NEW EUROPEAN PATENT SPECIFICATION

(11) **EP 0 994 716 B2**
(45) Date of publication and mention of the opposition decision: **04.07.2007**
(45) Mention of the grant of the patent: 11.02.2004
(21) Application number: 98936641.4
(22) Date of filing: 23.07.1998
(51) Int. Cl.: A61P 7/00, A61K 35/20, A23G 4/00, A61K 35/54, A61K 36/00

(54) **MATERNAL IMMUNE SECRETIONS AND THEIR USE IN THE TREATMENT AND/OR PROPHYLAXIS OF CONDITIONS OF THE HUMAN BODY**
MÜTTERLICHE IMMUNSEKRETIONE UND DEREN VERWENDUNG FÜR DIE BEHANDLUNG ODER PROPHYLAXE VON ZUSTÄNDEN VOM MENSCHLICHEN KÖRPER
SECRETIONS IMMUNES MATERNELLES ET LEUR UTILISATION DANS LE TRAITEMENT ET/OU LA PROPHYLAXIE D'ETATS DU CORPS HUMAIN

(30) Priority: 25.07.1997 IE 970541
(43) Date of publication of application: 26.04.2000
(73) Proprietor: Westgate Biological Limited, Donegal Town Co. Donegal (IE)
(72) Inventor: Folan, Michael Anthony, Donegal Town, Co. Donegal (IE)
(74) Representative: Duffy, Assumpta Dympna
(86) International application number: PCT/IE1998/000063
(87) International publication number: WO 1999/004804

(56) References cited:
- WO-A-88/02600
- WO-A-98/19558
- DE-A- 2 522 999
- FR-A- 2 182 659
- GB-A- 1 573 995
- IE-B- 65 218
- HEROD E.L.: "THE USE OF MILK AS A SALIVA SUBSTITUTE" J.PUB. HEALTH DENT., vol. 54, no. 3, 1994, pages 184-189, XP002084519
- HATTA H. ET AL.: "PASSIVE IMMUNIZATION AGAINST DENTAL PLAQUE FORMATION IN HUMANS: EFFECT OF A MOUTH RINSE CONTINGIN EGG YOLK ANTIBODIES (IgY) SPECIFIC TO STREPTOCOCCUS MUTANS" CARIES RESEARCH, vol. 31, no. 4, July 1997, pages 268-274, XP002084520 cited in the application
- SCHÜPBACH P. ET AL.: "INCORPORATION OF CASEINOGLYCOMACROPEPTIDE AND CASEINOPHOSPHOPEPTIDE INTO THE SALIVARY PELLICLE INHIBITS ADHERENCE OF MUTANS STREPTOCOCCI" J.DENT.RES., vol. 75, no. 10, October 1996, pages 1779-1788, XP002084521
- NAZARIAN L.F. ET AL.: "PRACTICE PARAMETER: THE MANAGEMENT OF ACUTE GASTROENTERITIS IN YOUNG CHILDREN" PEDIATRICS, vol. 97, no. 3, March 1996, pages 424-433, XP002084522

## Description

### Technical Field

This invention relates to the therapy and prophylaxis of conditions of the human body characterised by a diminution or loss of normal mucosal secretion flow or the normal protective properties thereof or to conditions of the human body which are responsive to such mucosal secretions.

By mucosal secretion herein is meant saliva and genital secretions.

### Background Art

Mucosal secretions have various properties, including mechanical lubrication and clearance, immunological protection, buffering capacity and re-mineralisation of the teeth when the mucosal secretion is saliva, and as solvents for neural stimulation.

Apart from hydrating and lubricating properties which are a feature of the mucopolysaccharides (mucins), saliva contains a number of specific and non-specific antimicrobial agents which regulate the microflora of the mouth.

Specific protection is provided by secretory immunoglobulins, which are produced by the immune system and targeted against cell surface antigens in response to particular microbial species. A range of biologically active molecules, some of which are poorly understood at present, provide more general protection; these include peptides and proteins that constitute the innate immune system. The enzymes, lysozyme, sialoperoxidase and amylase contribute to the non-specific antimicrobial action of saliva as do the agglutinating proteins and pellicle forming proteins, and lactoferrin all of which originate in the salivary secretions.

Lactoferrin is an iron binding protein, which effectively excludes iron from the nutrient supply of the oral microflora; it also modulates the availability of iron in the gastrointestinal (GI) tract and has a therapeutic function in controlling excessive absorption of this metal.

Saliva has a fundamental role in maintaining oral health. Any decrease in normal salivary flow or in its normal constituents can have a dramatic effect on the oral health of the individual and lead to increased incidence of Candida infection, gingivitis, periodontitis, dental caries, ulceration and halitosis. The relationship between the protective and preventative properties of saliva and the aforementioned infections is mainly prophylactic. Normal salivary flow in healthy individuals will not totally prevent the occurrence of any infection, but the reduction or loss of normal salivation will pre-dispose to greatly increased frequency and recurrence thereof.

The severity of any oral infection is also greatly influenced by the quantity and quality of an individual's saliva. Gingivitis and dental caries are considered among the most common diseases in the world; their ubiquitous nature resulting in most health authorities classifying them as hygiene or cosmetic related events. Yet in individuals suffering from severe loss of salivary function dental caries can become a rampant disfiguring and debilitating disease necessitating major dental and medical intervention.

The condition of loss of normal salivary gland function is medically described as xerostomia or 'dry mouth syndrome'. It is a very common condition caused by a wide range of factors. Many elderly people suffer from progressive xerostomia frequently caused by other medicinal substances such as anti-inflammatories, anti-histamines, analgesics, narcotics, antihypertensives, diuretics, psychotropics, antipsychotics, and anti-Parkinson drugs. Xerostomia is also a feature of Sjogren's syndrome, an auto-immune disease related to rheumatoid arthritis. Some of the worst affected individuals are those being treated by radiotherapy for head and neck cancers where destruction of salivary gland function as a result of radiation is an unavoidable side effect.

Although commonly described as 'dry mouth syndrome' xerostomia also frequently affects dryness of other areas of mucosa, particularly when it is a symptom of Sjorgen's Syndrome. Regardless of its aetiology. The condition has a serious impact on the quality of life of those affected and leads to an increased incidence of dental caries, gum disease, ulceration and thrush.

Xerostomia or any medical condition which results in debilitation of the mucosal secretion can pre-dispose the individual to greatly increased incidence of oral thrush caused by pathogenic yeast of the Candida species. Included among these pre-disposing conditions are immunosuppressive, cytotoxic and radiation therapy, HIV infection, leukaemia and diabetes. The least obvious and perhaps most prevalent pre-disposition to yeast infection arises from the over use of antibacterial drugs and steroids.

Oral thrush, or other manifestations of this infection such as yeast vaginitis are generically referred to as candidiasis; the most common species is *Candida albicans.* The emergence of new species of Candida from HIV infected individuals previously treated with long-term prophylactic antifungal drugs for oral candidiasis has been described by Coleman et al. AIDS (1997) 11, 5, 557 - 567.

The more ubiquitous oral healthcare problems include dental caries, gingivitis, periodontitis and halitosis discussed below. Most individuals will be affected by some or all of these at some point in their lives, the severity depending on a number of factors including host specific immune mechanisms, genetic factors affecting dental enamel and gum formation, dietary and hygiene factors affecting quantity and type of plaque microrganisms. While there is some conflict of opinion regarding the microbial aetiology of these infections, it is well known that specific microorganisms are involved in the progression of these afflictions. For example, there are three groups of microorganisms commonly associated with dental caries *namely, Streptococci, Lactobacilli and Actinomycetes.*

Many therapies exist for the treatment of conditions resulting from a diminution or loss of normal flow of a mucosal secretion or the normal immune properties thereof. For example, a wide range of antibiotics are used, including antifungal agents and antibacterial agents.

Antibiotics, whether they are antifungal or antibacterial, should be administered conservatively, as there is always a risk of resistance developing in the micro-organism being treated and of refractory infections. Most antibiotics have side effects which limit their use to acute treatments; these may range from nausea and GI upset to hepatic impairment and interaction with other drugs.

Pilocarpine, extracted from the plant *Pilocarpus microphilus*, used in the treatment of xerostomia is one of the most effective sialogogues available at present, its use requires individual dose monitoring as its side effects include lacrimation, dizziness, sweating, rhinitis and visual blurring. Pilocarpine has cholinergic activity at the muscarinic receptors and mimics the effect of acetylcholine from the parasympathetic nerves. Acetylcholine stimulates water and electrolyte secretions; a separate neurotransmitter, noradrenaline acts *via* the sympathetic nerves and stimulates protein and macromolecular secretion. In theory pilocarpine should only affect water and electrolyte secretion. There is, however, an amount of cross-reactivity at the cellular level where the common activator, cyclic AMP, affects both fluid and protein secretion, to a certain extent. The quality of the saliva or other mucosal secretion produced in response to pilocarpine is however limited in its content of essential macromolecules, particularly the secretory immune components. The secretory immunoglobulin content of saliva together with the other immune components are transported across the epithelium from the serum by a process of endocytosis and exocytosis, and arise mainly from the minor mucous glands, which are not directly affected by neurotransmitters.

It is also important to recognise that the use of pilocarpine to alleviate xerostomia is frequently indicated in individuals who have compromised immune systems due to other factors. Stimulation of fluid secretion in these patients cannot correct the inherent loss of the immune component.

It is a relatively simple matter to prepare a saturated solution of calcium and potassium salts that replicate the ionic constituents of saliva. Such a solution is already commercially available as "Luborant" (trade mark) from Antigen Pharmaceuticals Limited, consisting of potassium, magnesium and calcium chloride, with potassium phosphate buffers, sodium fluoride and carboxymethylcellulose. Similarly a number of re-mineralisation products have been developed including "Remodent" (trade mark) a solution of bone calcium. The problem with all of these products is that the delicate equilibrium of salivary saturation is not replicated by suitable crystal inhibitors which are active at the site of the caries lesion. The potential to form uncontrolled calculus deposition is therefore great.

Most humans are tolerant to egg protein as it is consumed regularly as a food, likewise most humans are tolerant to milk of various ruminant species.

It is known that the specific antibody content of milk and eggs can be amplified by prior immunisation of the cow or hen, respectively. Because of their food origin such antibodies provide an attractive option for long-term passive immunisation of the human mucosa. The potential use of hyperimmune egg immunoglobulin as a prophylactic against dental caries is described by Hatta, H et al: Caries Research, 1997:31:268-278.

EP-A 0 152 270 discloses a method of passively immunising a mammal against a condition caused by an antigen which involves administering to the mammal immunising amounts of an antibody obtained from a domesticated fowl or bovid which has been immunised against the antigen; the mammal being tolerant to the antibody by virtue of having a history of consumption of antibody-containing material derived from the egg of a fowl or milk of a bovid.

Irish Patent Specification No. 65218 describes the use of secretory immunoglobulin from eggs and milk as a prophylactic against fungal infections, particularly *Candida albicans* infections of the mucus epithelium. In this document it has been demonstrated that immunoglobulin fractions of eggs from hens immunised with *C. albicans* are effective in the inhibition of growth, buccal cell adhesion and hyphal tube formation of laboratory cultures of *C*. *albicans* species.

The use of egg immunoglobulin in passive immunisation is described further by Ikemori *et al.* (1992) American Journal of Veterinary Research; *53*, 2005. The role of secretory immunoglobulin and the potential for immunising against *Streptococcus mutans* was demonstrated by Mastecky et al., J. Clin. Invest. (1978) 61, 731-737.

By definition the process of passive immunisation is directed at specific microbial species and designed to eliminate these, without affecting other species that may be present. As described previously the buccal cavity in particular and the mucosa in general is subject to pathogenic colonisation by a wide range of microbial species. It is difficult to predict which, if any, or all, of the known pathogens will affect any one individual at a particular time, therefore passive immunisation techniques are at best, limited to prophylaxis and therapy of specific conditions.

The rationale for the use of passive immunisation in mucosal healthcare is based on the potential mimicry of the naturally occurring secretory immunoglobulins in the mucus. The presence of secretory immunoglobulin in human saliva is well known and suitably reviewed by Brandtzaeg in Human Saliva: Clinical Chemistry and Microbiology, Vol 2pp 1-55 Edited by J Tenovuo CRC Press 1989. In the same publication the non-immunoglobulin defence factors of human saliva are reviewed by J Tenovuo pp 55-93.

The non-immunoglobulin defence factors include lysosyme, lactoferrin and the sialoperoxidase enzymes as well as other innate immune system constituents. The innate immune system provides a broad-spectrum antimicrobiral activity without prior exposure to the antigen as is required in the specific, adaptive response, of which antibodies are the main component.

Others have reported the presence of innate immune components in human salivary secretions (Wolff et al., J.Histochem. Cytochem. (1990), 38, 1531 - 1534). Potent antimicrobial activity has been attributed to several groups of polypeptides isolated from mucosal secretions in many different vertebrate and invertebrate species, (Mahada, et al., Gut (1997), 40, 161-163). Among the identified molecular entities are the magainins, cecropins, defensins, collectins and proline rich and arginine rich peptides.

The potential use of antimicrobiral peptides in dental healthcare is the subject of a review by Miyasaki and Lehrer: Int' J. Antimicrobial Agents, 9 (1998) 269-280. The authors provide a comprehensive list of the currently known antimicrobiral peptides and their origin, none of which is obtained from milk or eggs.

There is a need for alternative and improved means of treating the aforementioned conditions of the human body by therapy or prophylaxis which obviate the side effects of conventional treatments.

### Disclosure of Invention

The invention provides use of those innate immune constituents of a maternal immune secretion selected from egg fluid and milk, which adhere to the surface of a microbial cell, said innate immune constituents being extracted from the maternal immune secretion, said extract having anti-microbial activity characteristic of a human mucosal secretion selected from saliva and genital secretion and which is tolerated by humans, in the manufacture of a medicament for the treatment and/or prophylaxis of a condition of the human body characterised by a loss of said mucosal secretion or the normal biological properties thereof or which is responsive to said mucosal secretion, the conditions of the human body being selected from a condition of the buccal cavity or vaginal infection, including yeast vaginitis.

The term human as used herein can embrace animals genetically closely related to humans and which would benefit from the therapeutic and prophylactic treatments described herein.

Maternal immune secretion as used herein means the biological secretion produced by the maternal parent of the species for the purpose of protecting the developing embryo or neonate, selected from egg fluid and milk, such as the fluid matrix of a hen's egg or the milk from a cow. Apart from their nutritive function, these fluids contribute passive immunity to the developing chick embryo or the intestine of the new-born calf by virtue of their secretory immune constituents, including, but not limited to antibodies.

The mucosal secretion is selected from saliva and genital secretion.

In the following description, the invention will principally be described with reference to a single maternal immune secretion, namely egg fluid material and to the use thereof in the treatment and/or prophylaxis of conditions of the buccal cavity.

Thus, in one aspect of the invention there is provided use of those innate immune constituents as defined above a maternal immune secretion having biological activity characteristic of human saliva and which is tolerated by humans in the manufacture of a medicament for the treatment and/or prophylaxis of a condition of the buccal cavity characterised by a loss of normal saliva or the normal biological properties thereof.

When the maternal immune secretion is egg fluid, then preferably the egg fluid is obtained from a domestic hen.

In one embodiment, the egg fluid is derived from egg yolk.

Preferably, the egg yolk is delipidised.

In an alternative embodiment, the egg fluid is derived from egg white.

The innate immune capacity of the egg is not restricted to the constituents of the egg yolk, egg white proteins exhibit anti-microbial activity which inhibit growth as well as adhesion and for this reason, these provide great utility in mimicking the characteristics of a mucosal secretion in accordance with the invention.

Preferably, the egg white is de-ovalbuminised egg white.

In order to utilise the antimicrobial value of egg white proteins it is preferable to remove the major structural protein ovalbumin. Methods of protein separation are well known to those skilled in the art. Any conventional method based on gel filtration, ion exchange, selective precipitation or other preparative chromatographic technique will suffice, as long as the residual minor constituents are recovered in a proportion approximating to their original ratio in the intact egg white.

In a further embodiment, the egg fluid is a mixture of delipidised yolk and de-ovalbuminised egg white.

Egg yolk extract and egg white extract as described herein provide a rich source of salivary and other mucosal secretion-like constituents including, but not limited to, egg lysozyme (lytic activity), ovotransferrin (iron binding), avidin (lectin and biotin complexing), ovomucoid (proteinase inhibition), mucin, lecithin and peroxidase activity as well as specific and non-specific immunoglobulins and non-specific anti-microbial components of the innate immune system.

Similar biologically active entities exist in the extracorporeal secretions of many vertebrates and particularly the extracorporeal glandular secretions of mammals, including the mammary gland. Extracorporeal secretions are biological fluids, which vitalise, invigorate and protect the interface between the body and the external environment. In the case of mammary secretions and the components of the avian egg, the extracorporeal secretions are also the maternal vehicle for transferring this interface to the neonate.

According to a still further embodiment, the secretion is caprine milk.

Equally milk from any ruminant will contain the expressed secretory immune components of the maternal animal. Colostrum, being the first milk after parturition, is particularly rich in immunoglobulin; it is naturally intended to provide passive immunity to the neonate prior to the maturity of the new-born's latent immune system. Like eggs, milk and particularly colostrum will contain secretory antibodies specific for any antigen to which the maternal animal has been exposed.

The choice of domestic eggs and milk from a cow or goat provides an acceptable source of these constituents. Because these materials are commonly consumed foodstuffs, they are not as universally unacceptable as for example donkey milk or pigeon eggs, although these would provide a completely adequate source of similar material for use in accordance with the invention.

As indicated above, these secretions are referred to herein as "maternal immune secretion". Extracts of such a secretion may be used to replicate the essential components of the human mucosal secretions defined above and can be used in accordance with the invention in the therapy and/or prophylaxis of conditions of the human body characterised *inter alia* by a diminution or loss of normal mucosal flow or the normal protective properties thereof.

The term prophylaxis as used herein embraces the use of maternal immune secretion in the manufacture of a medicament which can be used to amplify or substantially replace the normal mucosal secretion.

The medicament for use in accordance with the invention is a composition of biologically active ingredients of natural origin which mimics the effect of whole mucosal secretion, in particular by replacing the immune components, (both specific and non-specific), the lubricating and hydrating mucins and when required, in salivary replication the inhibitors of calcium phosphate crystallisation. This composition can replicate the known biological functions of whole mucosal secretions, using naturally occurring replicas of the macromolecular constituents thereof, as such it is suitable for long-term prophylactic administration in the healthcare applications described herein.

By medicament herein is meant also a special dietary formulation.

The innate immune system is distinguishable from the specific or adaptive immune system in that the components of the innate immune system are not specific to any one microbial species or antigenic type, nor do they require any prior exposure (immunisation) to elicit a response.

The maternal immune secretion can be amplified for specific applications by immunisation of the animal prior to collection of the secretion, however, this will not normally be required.

Thus, the maternal immune secretion or a portion thereof can be obtained from a hyperimmunised animal.

Preferably, when the medicament according to the present invention is a replica of saliva it will include a secretory immunoglobulin fraction as a constituent thereof, thereby mimicking the secretory IgA and IgG of human saliva. When desired the immune properties may be amplified by vaccinating the donor animal with antigens derived from any one or a combination of the following oral pathogens:
*Streptococcus* species; *mutans, rattus, cricetus, sobrinus, ferus, macacae, downei,* or serotypes thereof, *sanguis, oralis, mitis, salivarius, vestibularis.*
*Lactobacillus* species; *rhamnous*, *acidophilus* and *caseii*
*Actinomyces* species; *israellii, naeslundii* and *viscosus*
*Capnocytophaga* species; *sputigena ochracea* and gingivalis
*Fusobacterium nucleatum*
*Actinobacillus actinomycetemcomitans.*
*Candida* species; *albicans, dublinensis, glabrata, tropicalis krusei, kefyr* and *stellatoidae* among others.

As indicated above, in one embodiment of the invention the medicament is a replica of said human mucosal secretion.

In order to develop effective formulations for prophylaxis and therapy of the mucosa, it is necessary to replicate as closely as possible all the properties of the natural secretions. The most desirable formulations would include broad spectrum antimicrobial activity without total antisepsis, thereby eliminating excessive colonisation of opportunistic pathogens while facilitating the re-equilibrium of the natural microflora. Saliva naturally promotes growth of the normal microflora of the mouth, while at the same time inhibiting excessive colonisation of the mucosa. See, Saliva and Oral Health; Ed' Edgar & O'Mullane: published by British Dental Association; pages 100-101. The hydrating and lubricating properties of the natural secretions are fundamental in terms of healthcare of the mucosa, and in the case of saliva, the unique ability to regulate calcium crystallisation is critical to the maintenance of the tooth enamel. It is also desirable that formulations designed for long term prophylaxis are without the attendant side effects of conventional antibiotics or antiseptics.

Preferably, said secretion is enriched with one or more of the innate immune constituents thereof.

Thus, the present invention provides for the use of: i) combinations of innate immune system constituents which optionally include non-immunised antibodies; and ii) combinations of innate immune system constituents with immunised (hyperimmune) antibody; in maternal immune secretions or replicas thereof in the prophylaxis or therapy of the aforementioned conditions as hereinafter described in greater detail.

As described herein by extraction of the various innate immune constituents from maternal immune secretion and subsequent enrichment of maternal immune secretion or a replica thereof with the or each extracted immune system constituent one can potentiate the actions of the secretion or replica, as appropriate.

Typically the proteins of the innate immune system are extracted and enriched from the egg yolk in accordance with the invention based on their biological properties of adhesion to the surface of a microbial cell. Because of its use in food products, baker's yeast is suitable for use in this regard. The innate immune system constituents, which have affinity for a microbial cell, will adhere to the yeast and are recovered by centrifugation. By manipulating pH and ionic strength the innate immune system constituents can be desorbed from the yeast. Due to the polyspecific nature of the innate system, constituents from the fractions desorbed from baker's yeast will adhere strongly to a wide range of microbial species and in doing so will inhibit adhesion of these species to mucosal epithelial cells.

It is not necessary to remove the lipid fraction from the yolk, but doing so improves yield and facilitates measurement of the protein content. Where desirable egg yolk lipids can be removed using supercritical carbon dioxide to dissolve them or they can more simply be removed by diluting the egg yolk with nine volumes of distilled water and freezing at -70°C for 24 hours. On thawing, the lipid fraction will separate by centrifugation. The supernatant should be collected through a muslin cloth to remove low density lipid fractions which remain floating.

Microbial cells can effectively be used as a 'lure' for the innate immune system proteins. In this way, these proteins are enriched *en masse* using a procedure based on their biological property of adhesion to the surface of a microbial cell; this allows extraction and enrichment of all molecular entities sharing this property at the same time.

Apart from using microbial cells *per se,* it is also possible to use cell wall components of suitable microbial species bound to an inert surface such as polystyrene beads.

Egg yolk is a complex structure consisting of granules and spherical bodies suspended in an aqueous solution of protein (plasma), which is separated from the white by the vitelline membrane. The macromolecular constituents of the yolk consist of lipids, proteins, lipoproteins and phosphoproteins, oligosaccharides and glycoproteins, each with varying degrees of solubility in water and with varying degrees of affinity for each other so that some are aggregated in spherical bodies. A comprehensive review of egg proteins is available in; Egg Science and Technology, by Staedelman & Cotterill, published by Hayworth, ISBN # 156022 8555, pages 105-175.

The water soluble proteins of the egg yolk plasma have been classified as alpha, beta, gamma and delta livetins corresponding to serum proteins of the chicken. Chang, P.K. et al. (( 1970), J. Food Sci. 35, 87-88) reported that nineteen separate proteins were identifiable in lipid free extract of yolk while nine proteins were distinguishable in the alpha and beta fractions of the egg plasma; the gamma fraction was a single protein corresponding to serum gamma globulin. In 1969 Leslie, G.A. and Clem, L.W., (J. Exp. Med, 130, 1337-1352) identified the gamma globulin protein as immunoglobulin IgY.

Using isoelectric focusing (IEF) techniques the innate system constituents desorbed from bakers yeast can be shown to consist of up to eleven separate protein bands; two of these are heavily phosphorylated or glycosylated as they are only visualised with silver nitrate or amido black stains. At least one of the IEF bands corresponds to a gamma globulin (antibody) the others are non-immunoglobulin proteins.

As described previously the innate immune system capability is not restricted to constituents of egg yolk but includes also constituents of egg white. The individual constituents of the innate immune system may be fractionated by any means commonly utilised by those skilled in the art to provide purified components exhibiting discrete biological functions.

The purified fractions include proteins, lipoproteins, peptides, peptidoglycans, lipids, carbohydrates, oligosaccharides or any other macromolecular component or combination of components exhibiting biological functions similar to the properties of the mucosal secretion.

In the use of the innate immune constituents as described herein for the construction of synthetic mucous or for other prophylactic applications in the human mucosa, it may be desirable to standardise the relative concentration of each of the constituents. Standardisation can be accomplished by purifying quantities of each constituent and adding these back to a batch of de-ovalbuminised egg white, or de-lipidised egg yolk or extracts thereof.

Thus, the biological properties of constituents from eggs and milk which mimic the functions of saliva, and which can be extracted, purified and re-formulated to provide a replica of saliva are described in greater detail below, hereinafter also referred to as replica saliva.

Although not wishing to be bound by any theoretical explanation of the invention non-specific immune constituents such as oligosaccharides may potentiate the action of other constituents including immunoglobulins by conjugating individual components, thereby facilitating combined actions at the microbial cell surface.

The identified innate immune system of eggs includes the following entities which mimic similar factors in human saliva:
Lysozyme: Egg white is rich in the enzyme lysozyme and this constituent is easily extracted and purified. Saliva typically contains about 100 mg of lysozyme *per* litre. The antimicrobial function of this enzyme is well known to be the hydrolysis of murein in the bacterial cell wall, particularly in Gram positive bacteria including *Streptococcus mutans*, causing lysis of the cell in hypotonic solutions. Lysozyme is also thought to potentiate the action of secretory immunoglobulin.
Lactoferrin: Saliva contains 10-20 mg *per* litre of this iron binding protein which has been shown to sequestrate iron, denying it as a nutrient to the oral microflora. Egg white contains about 13% ovotransferrin which is now known to be structurally and functionally similar to salivary lactoferrin and human serum ferritin.
Growth factor inhibition: The presence of binding factors in saliva similar to lactoferrin but functional in sequestrating Vitamin B12 has been suggested by Tenovuo, J.O. ((1989) *supra*). Egg white contains avidin which is known to bind biotin to such an extent that it can cause nutritional deficiency of this vitamin in rats. Biotin is an essential growth factor for *Candida albicans* and other pathogenic yeast species, (F.C. Odds; Candida and Candidosis. Page 13).
Enzyme inhibition: Saliva is also known to contain a range of enzyme inhibitors. Thus, saliva has been shown to contain cystatins, inhibitors of bacterial protease activity (Saitoh. E. and Isemura S. Crit. Rev. Oral Biol. Med. (1993) 4, 487-493). As well as cystatins, egg white contains trypsin inhibitors known as ovomucoid, which exhibit powerful proteolytic inhibition not dissimilar to the cystatins and other salivary constituents.
The Livetins: Non-specific and polyspecific constituents of egg yolk including gamma livetin which act in a manner similar to secretory antibodies by binding to the surface of microbial cells thereby creating a molecular barrier to prevent adhesion to the host tissue.

The replicated properties of saliva, and other mucosal secretions, as appropriate as described herein, include, but are not restricted to:
The innate immune properties of lysozyme, sialoperoxidase, histatins, mucins, adhesins, defensins, magainins, collectins and cecropins;
The proteolytic inhibition of ovomucoid and other factors;
The metabolic regulation of lactoferrin, avidin and other growth factor sequestrating agents;
The calcium crystal inhibition and pellicle forming properties of statherins, proline rich proteins (PRP) and phosphoproteins;
The hydrating and lubricating properties of mucins and glycoproteins;
The re-mineralisation properties of calcium carbonate;
The buffering properties of calcium and potassium phosphate; and
The microbial nutrient properties of, glucose, urea and free amino acids.

Replica saliva can be amplified in accordance with the invention for the prophylaxis and therapy of oral infection.

Specific formulations can be prepared by increasing the concentration of individual constituents to target specific microorganisms.

Also specific immunoglobulins derived from animals hyperimmunised with suitable antigens can be combined with the innate system constituents extracted as described herein.

Anti-candida formulations in accordance with the invention will typically include combinations of specific anti-candida immunoglobulins together with an increased concentration of avidin. The avidin in such formulations will bind biotin, an essential growth factor for Candida. Increasing the concentration of ovotransferrin in such formulations to sequestrate iron will deprive the yeast of both iron and biotin, thereby resulting in greatly increased antimicrobial activity.

It will be appreciated that such formulations will be effective in the treatment of Candidiasis generally and especially vaginal thrush.

Anti-halitosis formulations in accordance with the invention will typically include combinations of specific immunoglobulins directed against the Gram negative anaerobic organisms commonly associated with proteolytic activity in the mouth including *Treponema denticola, Porphyromonas gingivalis, Prevotella intermedia, Bacteroides forsythus* and *Fusobacterium* and ovomucoid, which inhibits the proteolytic activity of these organisms.

Increasing the concentration of lysozyme in the above formulations will potentiate the antimicrobial activity further, even for Gram negative organisms which are not usually affected by lysozyme on its own.

Anti-plaque formulations in accordance with the invention will typically include combinations of immunoglobulin specific for *Streptococcus mutans* species together with an increased concentration of lysozyme, which has potent lytic activity against these Gram positive cocci.

By increased concentration of each of the above identified individual constituents of the replica saliva is meant an amount of said constituent which is increased relative to the amount existing in the replica saliva by adding a purified extract thereof to a batch of replica saliva. Preferably, the amount of avidin added would not be greater than 1mg/ml and the amount of lysozyme or ovotransferrin added would not be greater than 5mg/ml.

In the above formulations it is preferable, although not essential, that the composition be prepared in a dilute solution of aqueous extract of whole egg.

It is found that individual constituents which exhibit characteristic biological activity are potentiated by dilute aqueous extract of whole egg. Although not wishing to be bound by any theoretical explanation in this regard, it is proposed that certain water soluble components act as aggregating factors facilitating conjugation of the individual components at the microbial cell surface.

The constituents of replica saliva in accordance with the invention, being typically constituents of domestic eggs, which have been extracted, purified and re-assembled in a formulation with specific features depending on their intended use, will be in the form best suited to the application. The active ingredients can be in the form of a freeze-dried powder designed for inclusion in toothpaste, or they may be assembled in liquid form intended as a mouthwash. They may also be encapsulated or emulsified in a wax or polymer for use as a coating of dental floss or as a fixative for dentures. Other suitable formulations include denture liners and pastilles.

In any preparation for use in accordance with the invention the various constituents can be combined with excipients designed to enhance the adhesion of the active constituents to the oral or other mucosa or to provide sustained or controlled release of the constituents at the intended site of action.

In a preferred embodiment for oral application the formulation of active ingredients is presented in a sustained release formulation of chewing gum.

Medicaments prepared from maternal immune secretion in accordance with the invention can be used in a wide range of conditions and diseases of the buccal cavity.

Formulations of replica saliva for use in accordance with the invention can be used for general prophylaxis against dental caries and gum disease, in individuals with no pre-disposition to other oral disease, or they can be more specifically tailored to complement normal salivary flow against recurring infections such as thrush in otherwise healthy individuals. The replica saliva can also be constructed so as to totally replace the debilitated immune content of normal saliva in immuncompromised individuals. Replica saliva can also be formulated so as to supplement or totally replace debilitated salivary gland function in xerostomia. Even more specific formulations are possible involving individual components such as the protease inhibitors (ovomucoid) for bacterial halitosis.

As indicated above, the medicaments described herein can be used in the treatment and/or prophylaxis of oral infection.

For example, the medicaments can be used in the prophylaxis and therapy of oral thrush, which may be a recurring infection caused by xerostomia.

The medicament according to the invention can also be used in the prophylaxis of dental caries.

The medicaments described herein can also be used in the treatment and/or prophylaxis of a condition of the buccal cavity characterised by inflammation of the gums.

A further use for the medicaments described herein in accordance with the invention is in the treatment and/or prophylaxis of halitosis.

In the case of oral xerostomia and halitosis preferred formulations are mouthwashes and chewing gum as indicated above.

Saliva is a masticatory lubricant and so bathes all food which is chewed. The role of salivary amylase is well documented in that it is commonly held to initiate digestion of starch in the mouth. Less well understood is the interaction between other salivary constituents and food, particularly in the modulation of absorption of food ingredients.

Thus the iron binding properties of ovotransferrin and phosvitin can be used to sequestrate iron in the diet, thereby limiting its availability for systemic absorption as may be desirable in the treatment of haemochromatosis. The addition of the proteolytic inhibitor, ovomucoid, to such formulations will further inhibit the trypsin digestion of the ovotransferrin:iron complex, thereby further limiting the dietary uptake of this element.

Similarly there would appear to be a function involving secretory IgA in regulating the uptake of allergens from food. The medicaments in accordance with the invention can be used to inhibit the uptake of constituents of food, which may cause allergies in some individuals. By immunising the donor animals against specific allergens the immunoglobulin content can be directed against these constituents thereby minimising the absorption of these allergens from the gut.

Thus, it is possible to use the medicaments in accordance with the invention to affect the uptake of particular constituents from food.

Furthermore, the medicaments can comprise one or more inorganic salts characteristic of human saliva.

The therapeutic properties of replica saliva in accordance with the invention can be amplified still further by the incorporation of herbal extracts known to have restorative properties in oral healthcare.

Thus, the medicaments described herein in accordance with the invention can be administered simultaneously, separately or sequentially with a plant extract which potentiates the effect of said secretion.

Suitably, the plant extract is obtained from *Vaccinium myrtilis*, *Zea mais, Melissa officianalis* or *Pilocarpus microphilus.*

Scientific literature and pharmacopoeial references support the therapeutic value of *V. myrtilis* extract in oral healthcare: Morazzoni, P. and Bombardelli, E., Fitoterapia (1996) LXVII, 1.

The anthocyanins found therein provide the following features:
Stimulation of biosynthesis of mucopolysaccharides
Stimulation of biosynthesis of glycosaminoglycans
Anti-inflammatory effect due to reduction in capillary permeability probably as a result of cross-linking collagen thereby increasing resistance to collagenase activity.

The general astringent properties of the standardised extract causes increased salivation and mucous secretion in the mouth.

Rosmarinic acid from *Melissa officianalis* (lemon balm) has been clearly demonstrated to have therapeutic value in the treatment of *Herpes simplex:* Wolbling, R.H. and Leonhardt, K., Phytomedicine (1994) 1, 25-31.

Insaponifiable fraction of *Zea mais* is reported to stimulate alveolar bone growth by stimulating the osteoblast cells, and is consequently valuable in the repair of periodontal bone loss. (Nuova Linnea Sa, Locarno, Switzerland).

The drug pilocarpine is normally administered to those suffering from xerostomia in capsule form, 5mg three times daily. The drug is a cholinergic agonist and affects increased fluid production with little improvement in the macromolecular constituents which are essential to the biological function of saliva. The side effects from this therapy are frequent and include sweating, lacrimation and nausea. In order to minimise these side effects it is common to tailor the dose to the individual; the individually effective dose may vary from 1mg to 15mg three times daily.

The plant was known to the South American Indians as Joborandi, it was commonly chewed by them to avail of its sialogenic properties. Incorporating pilocapine in chewing gum will facilitate individual dose monitoring in the same way as this is facilitated by those using nicotine gum as a smoking substitute. The inclusion of the extracts of the maternal immune system secretions described herein together with pilocarpine in chewing gum will provide increased fluid and macromolecular constituents necessary to replicate normal saliva.

In a further aspect, the invention provides a formulation for use in the treatment and/or prophylaxis of a condition of the buccal cavity characterised by a loss of normal saliva or the normal biological properties thereof which comprises a maternal immune secretion as hereinbefore defined and a polyol selected from erythritol, mannitol, sorbitol and xylitol.

Such polyols are known for use in dental hygiene products and interfere with microbial conversion of dietary carbohydrate and so prevent the formation of acid in dental plaque.

Further, preferably, the formulation is in the form of a chewing gum, pastille, mouthwash or a toothpaste.

Medicaments for use in accordance with the invention can be administered or applied in any form effective to achieve the desired therapeutic or prophylactic effect.

The formulations described above are typically those suitable for treatment of the buccal cavity. However, it will be appreciated that other applications will include formulations for oral administration, parenteral administration and topical application, nasal sprays, pessaries and suppositories.

### Brief Description of the Drawings

Fig. 1 is a graph showing the number of *C. Albicans* cells which adhere to buccal epithelial cells in the presence of enriched egg-yolk protein alone or in combination with de-ovalbuminised egg white relative to hyperimmune anti-Candida immunoglobulin and a control as described in Example 6;
Fig. 2 is a graph of O.D. at 600nm *versus* dose of de-ovalbuminised egg white (mg/ml) in culture media at 9 hours after innoculation as described in Example 7;
Fig. 3 is a graph of O.D. at 600nm *versus* dose of de-ovalbuminised egg white (mg/ml) culture media at 9, 12 and 15 hours after inoculation as described in Example 7.
Fig. 4 is a graph of O.D. at 600nm *versus* time (hours) showing growth of *C. Albicans* on yeast nitrogen base with 2% glucose supplemented with lysozyme (1mg/ml), ovotransferrin (1mg/ml) or avidin (0.1 mg/ml) or combinations thereof as described in Example 7;
Fig. 5 is a graph of O.D. at 600nm *versus* time (hours) showing growth of *C. Albicans* on Yeast Nitrogen Base with 2% glucose supplemented with de-ovalbuminised egg (1mg/ml) or with a combination of ovotransferrin (0.25mg/ml), lysozyme (0.1 mg/ml) and avidin (0.05mg/ml);
Fig. 6 is a composite graph representing loss of calcium from a saturated solution (% of saturated solution) *versus* time (minutes) in the presence of saliva, innate immune constituents of egg and a blank as described in Example 9;
Fig. 7 represents a series of graphs of O.D. at 600mn *versus* time (hours) showing growth of *St. mutans* in 80% SBNB with varying concentrations of xylitol and innate immune constituents of egg as described in Example 10;
Fig. 8 is a series of bar graphs of O.D. at 600mn *versus* increasing concentration of de-ovalbuminised egg (mg/ml), and de-ovalbuminised egg in the presence of xylitol (0.5%) as described in Example 10;
Fig. 9 represents a series of graphs of O.D. at 600mn *versus* time (hours) showing growth of *St. mutans* in 80% SBNB in the presence of various polyols (0.5%) and innate immune system extract of egg (0.075%) as described in Example 10; and
Fig. 10 shows the relative % adhesion of *St. mutans* to hydroxyapatite beads in the presence of innate immune system proteins from eggs and milk compared with hyperimmune anti-*St. mutans* egg IgY as described in Example 12.

The invention will be further illustrated by the following Examples:

### Modes for Carrying out the Invention

### Example 1

This Example describes how the aqueous fraction of whole egg will inhibit the binding of *Streptococcus mutans* to saliva saturated, experimental dental enamel (hydroxyapatite beads). Whether this is because of competitive binding with the pellicle layer or because of specific inhibition is not yet known.

Approximately 100 ml of saliva was collected over ice from one individual during a two hour period; salivary flow was stimulated by chewing a piece of 'Parafilm' (Parafilm is a trade mark).

The egg extract was prepared by breaking one egg into a litre of sterile distilled water, mixing this by shaking for one minute and allowing it to stand in the fridge for 12 hours. The aqueous soluble fraction was decanted from the insoluble precipitate and used as follows. One half of the saliva sample was diluted with physiological saline, the other half was diluted with the aqueous fraction of non-specific whole egg. Both fractions were sterile filtered using a 0.2 micron filter.

To each 50 ml fraction, 1 gram of sterile hydroxyapatite beads (BDH) was added and allowed to hydrate for a period of 12 hours. Both fractions were inoculated with a suspension of *Streptococcus mutans* culture grown on sucrose blood agar under carbon dioxide enriched nitrogen (to encourage extracellular polysaccharide production) and incubated for twelve hours at 30°C .

The beads from both samples were washed twice with equal volumes of sterile physiological saline and stained using the standard Gram stain procedure. While the hydroxyapatite beads take up some of the dye, the Gram positive cocci on the beads are clearly distinguishable under the microscope. Using this subjective analysis it is evident that the egg yolk extract inhibits adhesion of the *St. mutans*, as there were approximately 100 times greater numbers of cells visible on the beads from saliva only.

The fact that aqueous extract from non-immunised eggs showed inhibition of *St. mutans* adhesion to hydroxyapatite in the presence of salivary proteins requires further investigation. It is possible that the eggs contain background immunoglobulin to streptococcal species due to environmental exposure of the chickens to this organism. Alternatively the components of the egg yolk which are shown below to inhibit calcium precipitation may also bind to the statherin and PRP components of saliva creating steric hindrance to further binding by *St. mutans.* It is also possible that the innate immune components of the eggs contribute to the inhibition of adhesion.

### Example 2

This Example demonstrates that the aqueous soluble fraction of whole egg has the effect of inhibiting calcium precipitation.

The aqueous soluble extract of whole egg was prepared as described in Example 1. The temperature of the egg solution was maintained by standing the solution in a water bath at 20°C. Calcium bicarbonate was added to the crude solution of egg until a saturated solution was obtained; a precipitate formed which combined with undissolved calcium carbonate at the bottom of the flask. After 12 hours the clear supernatant was decanted into a fresh flask and maintained at 20°C in the water bath. A similar solution was prepared using distilled water and calcium carbonate without the egg, the supernatant being similarly decanted from the undissolved crystals. The two solutions were placed in open topped 500 ml beakers with 500 mg of hydroxyapatite beads (BDH), as crystal initiators, in each. The solutions were kept in the water bath at 20°C for three days, and the rate of crystallisation observed over that period. Crystallisation was apparent in the solution free of egg after 24 hours, and did not appear in the egg solution until the sixtieth hour. The experiment was repeated three times with similar results.

Exactly why egg should have crystal inhibition properties is not known. It is possible to speculate that this is a biological mechanism, which is applicable in many different roles including calcium deposition in the shell or in the developing chick embryo. For whichever reason the feature exists, it lends itself to serendipitous use in formulating salivary replacement products.

### Example 3

### Replica of human saliva designed as a general prophylactic in oral healthcare

The yolk of egg from non-immunised hens is used as the starting material for the above purpose in this Example. The entire aqueous soluble fraction is purified by removing the cholesterol fraction only. This is accomplished by any one of the industry standard techniques; the use of liquid carbon dioxide is gaining acceptance in the food industry and is a very suitable method for removing lipids and fat soluble fractions from an aqueous solution of egg yolk without harming the protein fraction. The residual water soluble fraction which contains the immunoglobulin fractions and the non-specific immune constituents, and other active ingredients is freeze dried.

The freeze dried crude extract may be incorporated into any suitable delivery system including chewing gum and used to give a general prophylactic benefit in oral healthcare as hereinbefore described.

### Example 4

### Saliva replica designed for general prophylaxis in oral healthcare

Domestic hens may be immunised using any one or a combination of microbial antigens indicated previously, or any antigen implicated in disease due to oral absorption. When the egg antibody titre has been maximised the eggs are collected and the immune constituents purified.

Using industry standard protein fractionation techniques, including gel filtration chromatography, the constituents of the watersoluble fraction of white of egg can be separated. Specific fractions containing lysozyme, ovotransferrin, avidin and ovomucoid can be further purified using ion exchange chromatography and molecular sieve techniques. Specific glycoprotein fractions can also be isolated, these contain the mucins and agglutinating proteins.

Fractions demonstrating calcium crystal inhibition have been isolated from both yolk and white, indicating either that these are separate molecular entities with similar properties, or that they are constituents of the vitelline membrane.

Each separate fraction is freeze dried and characterised according to protein content and specific activity. The purified fractions can be used to prepare a standardised formulation.

A solution of the following fractions is then prepared in sterile aqueous extract of whole egg.

| Constituent | Approximate Concentration |
|---|---|
| Non-specific immune constituents | 2000 mM |
| Specific immunoglobulin | 200 mM |
| Glycoproteins | 200 mM |
| Lipopolysaccharides | 200 mM |
| Mucins | 200 mM |
| Ovomucoid | 100 mM |
| Lysozyme | 100 mM |
| Avidin | 100 mM |
| Ovotransferrin | 100 mM |
| Urea | 10 mM |
| Essential amino acids | 10 mM |
| Essential fatty acids | 10 mM |
| Calcium | 2 mM |
| Magnesium | 0.2 mM |
| Sodium | 20 mM |
| Potassium | 20 mM |
| Ammonia | 5 mM |
| Hydrogen phosphate (divalent) | 20mM |
| Dihydrogen phosphate (monovalent) | 20mM |
| Bicarbonate | 20mM |

The pH of the solution is titrated to 6.7 using 0.1 Molar hydrochloric acid or 0.1 Molar ammonium hydroxide.

The solution is freeze dried and used as an active ingredient in the preparation of final formulations of chewing gum, pastilles, toothpaste, dental floss, denture liners and mouthwashes, it may also be incorporated into special dietary formulations.

### Example 5

### Extraction and enrichment of innate immune system constituents from egg yolk

Fresh baker's yeast (*Saccharomyces cerevisiae*) was inactivated by suspending 40g in 1 litre of 10mM phosphate buffer pH 7.0 and heating to 60°C for one hour. Inactivation is necessary to prevent protease activity during the extraction procedure. The use of baker's yeast is favoured because it is a food constituent and therefore gives no cause for concern as regards safety or toxicology. The inactivated yeast was then prepared by centrifugal washing first in 1.0 M potassium phosphate buffer at pH 4.0 containing 0.01% Tween 80 (Tween is a Trade Mark) followed by two washings in 10mM potassium phosphate buffer pH 7.0. The effect of the high ionic strength, low pH buffer with Tween is to remove any loosely adhering capsular protein or cell surface macromolecule which may interfere with the extraction process.

A lipid free extract of egg yolk was prepared by a method hereinabove described and the protein concentration thereof was adjusted to 10mg/ml using 10mM potassium phosphate buffer at pH 7.

The prepared yeast was added to the egg yolk solution in the amount of 1 gram wet weight of cells to each 25 mg of protein. The suspension was incubated at 30°C for one hour during which the innate immune system constituents bound to the surface of the yeast cell. The yeast together with the bound material was recovered by centrifugation and washed twice in 10mM phosphate buffer at pH 7.0, re-suspended in 1.0 M potassium phosphate buffer at pH 4.0 containing 0.01% Tween, and agitated vigorously for 30 minutes to desorb the bound egg constituents. The spent yeast was removed by centrifugation and the supernatant dialysed against 10mM potassium phosphate buffer at pH 7.0.

Proteins were determined herein by the Lowry assay using bovine serum albumin as a standard.

The yield of recovered protein was 48% of the protein content of the egg solution. Marginally higher yields can be achieved by repeating the process with fresh yeast in the same egg yolk solution or by desorbing at lower pH. The maximum recovery obtained using the methods described was 54%, indicating that some 40% of the egg yolk protein does not bind to the yeast cell.

Using isoelectric focusing on 4% polyacrylamide gel (Pharmacia, Sweden) the protein profile of the de-lipidised egg was compared with the desorbed fraction from baker's yeast. Sample material was applied to the gel using a micropipette (50µg protein/20µl) and the gels were run under non-reducing conditions using a BioRad (BioRad is a Trade Mark) power pack at 100 watts in conventional manner. Using a combination of coomassie blue and silver nitrate stains, nineteen separate proteins in the de-lipidised egg fraction were visible; this corresponds-to the report of Chang, P. K. *et al*. ((1970) *supra*). Nine bands were visible in the desorbed (enriched) protein fraction, with two other minor bands visible only with silver nitrate staining corresponding to acidic phosphoproteins. It is important to note that the number of protein bands cannot be equated directly to the total amount of protein enriched from the egg solution, as some of the apparent protein bands may be subunits of individual globular proteins which dissociate due to the desorption process.

### Example 6

### In vitro efficacy of enriched egg yolk constituents

A significant feature of the enriched egg protein prepared in Example 5 is its ability to bind to a wide range of microbial species. In order to demonstrate the polyspecific binding capacity of the enriched egg yolk proteins, heat inactivated suspensions of the following organisms were prepared in 10mM potassium phosphate buffer pH 7.0: *Saccharomyces cerevisiae, Candida albicans, Streptococcus mutans, Streptococcus faecalis, Lactobacillus casei, Porphyromonas gingivalis, Staphylococcus aureus, Salmonella typhimurium, Clostridium difficile* and *Pseudomonas aeruginosa.* Broth cultures of the organisms were grown as described below, washed by centrifugation using high ionic strength buffer followed by equilibration at low strength buffer and diluted to an Optical Density at 600nm of 1.0.

### Microbial Cell Culture:

The yeast *Candida albicans* was cultured on Sabouraud Dextrose agar from Oxoid. Broth cultures were prepared in Sabouraud Dextrose broth, when cells were required for buccal cell adhesion assay or for evaluation of the anti-adhesive properties herein of any of the innate immune system extracts herein. Inhibition of growth was evaluated in Yeast Nitrogen Base from Difco supplemented with 2% glucose.

The bacteria, *Streptococcus mutans, Streptococcus faecalis, Porphyromonas gingivalis, Staphylococcus aureus, Salmonella typhimurium* and *Clostridium difficile* were cultured on Columbia blood agar base from Oxoid supplemented with de-fibrogenated horse blood from oxoid. *Clostridium difficile* and *Porphyromonas gingivalis* are obligate anaerobes and were cultured under anaerobic conditions in a 'gas jar' using anaerobic gas packs from Oxoid. When anti-adhesion studies were being conducted with *Streptococcus mutans* the organism was first cultured on Brain Heart Infusion Broth under anaerobic conditions in a gas jar to encourage the formation of cell surface glycans which are thought to contribute to the adhesion of this organism.

5.0 ml of each heat inactivated culture was added to 5.0 ml of enriched egg protein solution containing 10 mg *per* ml total protein and incubated at 30°C for one hour. The cultures were heat inactivated in the manner described in Example 5. The microbial cells were recovered by centrifugation, washed and the bound protein desorbed in the manner described in Example 5. The results of the proteins bound to each cell as a measure of residual protein in the supernatant after incubation and the amount recovered by desorption are shown in Table 1.

**Table 1**

| Adsorption and Desorption of Enriched Egg Yolk proteins from a series of organisms | | | | | | |
|---|---|---|---|---|---|---|
| Organism | Total Applied | Residue in supernatant | Wash | Amount adsorbed | Amount desorbed | Desorption + wash + residue from supernatant |
| *S. cerevisiae* | 50mg | 1.8 | 2.9 | 45.3 | 32.5 | 37.2 |
| *C. albicans* | 50mg | 2.1 | 0.1 | 47.8 | 35.6 | 37.8 |
| *St. mutans* | 50mg | 3.5 | 2.4 | 44.1 | 26.5 | 32.4 |
| *St. faecalis* | 50mg | 3.3 | 1.9 | 44.8 | 31.6 | 36.8 |
| *Lac. casei* | 50mg | 3.8 | 2.3 | 43.9 | 32.8 | 38.9 |
| *P. gingivalis* | 50mg | 5.3 | 1.7 | 43.0 | 33.6 | 40.6 |
| *Staph. Aureus* | 50mg | 2.0 | 1.5 | 46.5 | 40.3 | 43.8 |
| S. *typhimurium* | 50mg | 2.8 | 1.5 | 45.7 | 34.5 | 38.8 |
| *C. difficile* | 50mg | 6.5 | 1.9 | 41.6 | 31.4 | 39.8 |
| *Ps*. *Aeruginosa* | 50mg | 2.4 | 2.1 | 45.5 | 30.6 | 35.1 |

In each case approximately 15%-20% of the applied protein was found to be missing, the most likely explanation being that it was still bound to the microbial cells.

### Buccal Epithelial cell Adhesion:

In order to demonstrate the efficacy of the enriched egg yolk *proteins in vitro* buccal cell adhesion studies were conducted in the following manner.

Buccal epithelial cells were collected from the inside of the cheek using a 'Costar' cell scraper (or with the sharp edge of a spoon). The cells were diluted in phosphate buffered saline at pH 7 and washed through an 18 micron filter cloth to remove cell debris and bacteria. Washed cells were counted using a haemocytometer slide under a microscope. The cells were mixed with freshly grown (washed) yeast in the ratio of 100 yeast to each buccal cell and incubated with or without test material at 30°C for 1 hour with gentle agitation. Where 'Cytospin' (Cytospin is a Trade Mark) agitators were used these were set to rotate at 200 r.p.m..

After incubation the cell mixture was filtered through 18 micron filter cloth and transferred to a clean microscope slide and allowed to air dry overnight. The dried slides were 'heat fixed' by passing them briefly through a bunsen flame. An aqueous solution of crystal violet (1% w/v) was applied to the slide for one minute rinsed and allowed to dry. Under microscopic examination at x 500 the number of yeast cells adhering to each buccal cell was relatively easy to count. The average number of yeast cells adhering to 100 buccal cells was taken as a measure of the adherence factor.

The enriched egg yolk proteins were compared with hyperimmune anti-candida immunoglobulin extracted from eggs laid by hens immunised with heat killed *C. albicans* cells. Hyperimmune anti-Candida immunoglobulin was prepared as follows.

### Preparation of hyperimmune antibody

Freshly grown *C*. *albicans* cells were diluted in phosphate buffered saline to a concentration giving an optical density of 1 at 600nm. The cell suspensions were attenuated by heating to 60°C for one hour, and used immediately.

Egg laying chickens were injected at two sites intra-muscularly with 1 ml of attenuated cells in Freund's complete adjuvant at two weekly intervals over a period of eight weeks. The antibody titre was measured using an ELISA technique with peroxidase conjugated goat anti-chicken immunoglobulin (Sigma, England).

Hyperimmune egg antibody was isolated from the egg as follows: the egg yolk was separated from the white onto a clean tissue to dry any residual thin white from the external surface of the viteline membrane. Collected yolks were disrupted in a glass beaker and 9 volumes of distilled water added. The solution was frozen at -70°C overnight. On thawing the solution was centrifuged and the supernatant decanted through muslin to remove low density lipid which remains floating.

12% polyethylene glycol (molecular weight 8,000) was added in a step-wise fashion to the de-lipidised egg and allowed to equilibrate at room temperature for one hour. The precipitate was recovered by centrifugation and made up to its original volume with ice-cold ethanol, after 20 minutes the solution was centrifuged and the sediment re-suspended in a minimum volume of 10mM phosphate buffer. The dissolved fraction was dialysed against phosphate buffer overnight.

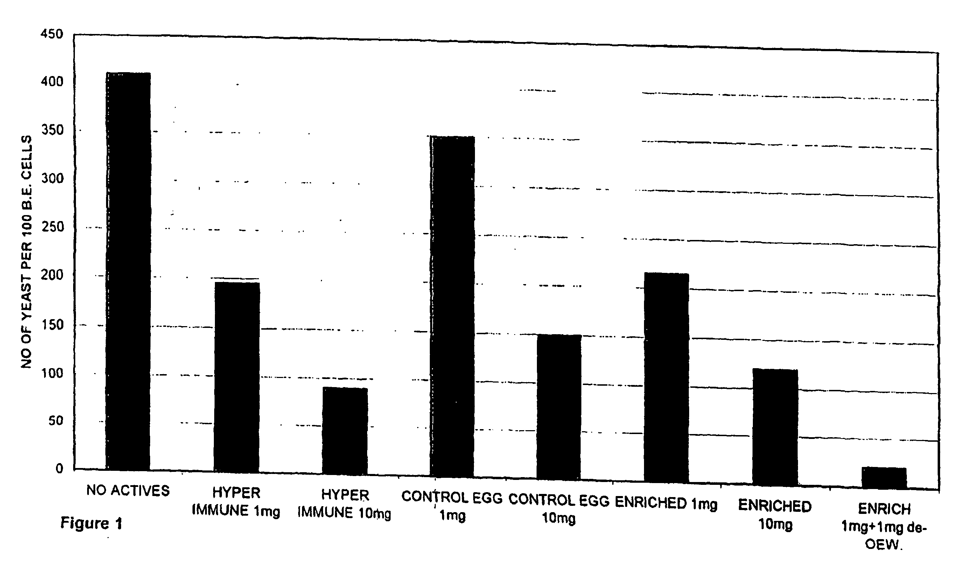

Control egg was de-lipidised egg yolk which had not been enriched, all three fractions were tested at 1.0 mg *per* ml and at 10 mg *per* ml. The results are shown in Fig. 1.

Fig. 1 shows that hyperimmune egg immunoglobulin at 10 mg *per* ml effects a 79% reduction in the number of yeast adhering per 100 buccal cells. 10 mg *per* ml of enriched egg proteins achieves a 70% reduction, while 95% reduction is achieved by combining 1.0 mg *per* ml of the enriched egg proteins with 1.0 mg *per* ml de-ovalbuminised egg white (DeOEW), obtained as described in Example 7.

### Example 7

De-ovalbuminised (de-OEW) egg was prepared as follows:

An aqueous solution of fresh egg white in 9 parts of de-ionised water was prepared and ammonium sulphate was added to a final concentration of 90% w/v. After equilibration for 30 minutes the protein precipitate was recovered by centrifugation and re-suspended in de-ionised water. The re-dissolved precipitate was dialysed against 10mM ammonium acetate buffer at pH 5.5 and adjusted to 10mg of protein *per* ml using the same buffer.

The prepared egg white solution was applied to a column of Whatman DE-52 (Whatman is a Trade Mark) resin previously equilibrated with 10mM ammonium acetate at pH 5.5 at 10mg *per* ml of column packing. Under these conditions, lysozyme, avidin and ovotransferrin did not bind to the resin and were recovered in the initial elution from the column. Using a gradient of increasing ionic strength (10mM to 500mM ammonium acetate), cystatin, ovoinhibitor, G2 and G3 globulins were removed in the eluent. Because of similarity in iso-electric points the late eluent from this column contained mixed fractions of ovomucoid, ovomucin, ovoglycoprotein, ovoflavoprotein and ovomacroglobulin in ovalbumin.

The late eluent from the DE-52 column was concentrated by ultra-filtration using a 10,000 Kda exclusion membrane and then applied to an S-100 gel filtration column using 10mM ammonium acetate pH 5.5 buffer as the carrier. Based on molecular weight separation the initial eluent contained ovomucin and ovomacroglobulin which was followed by a large fraction containing ovalbumin, thereafter ovomucoid, ovoflavoprotein and ovoglycoprotein were recovered in the late eluent.

The protein recovered from the S-100 column before and after elution of the ovalbumin was combined with the material eluted from the DE-52 column prior to the appearance of the mixed ovalbumin fraction. The combined solutions were concentrated by ultra-filtration and dialysed in the UF cartridge by adding 10mM potassium phosphate buffer at pH 7.0.

De-ovalbuminised egg white was found in this Example to exhibit a unique dose response relationship when added to cultures of bacteria or yeast. The inhibitory activity of this material is affected by the strength of the culture media, the age of the inoculum and the dose. Unlike most inhibitory substances the effect does not increase with increasing dose; there is an inverse response at concentrations above 1.0 mg *per* ml where the material stimulates growth. Between 1.0 and 0.1 mg *per* ml (depending on media strength) there is inhibition of growth. This unique dose response may be a feature of the solubility of the de-OEW, or the solubility of individual constituents therein. Saliva is known to exhibit similar growth stimulation and inhibition and this is considered to be one of the pre-requisites for maintenance of a normal microflora in the mouth: See, Saliva and Oral Health; Ed' Edgar & O'Mullane; Published by British Dental Association; pages 100-101. The long-term use of antibiotics and bactericidal agents results in total antisepsis of the mouth, eliminating the healthy microflora which saliva naturally supports, so supporting further opportunity for subsequent colonisation by inappropriate species.

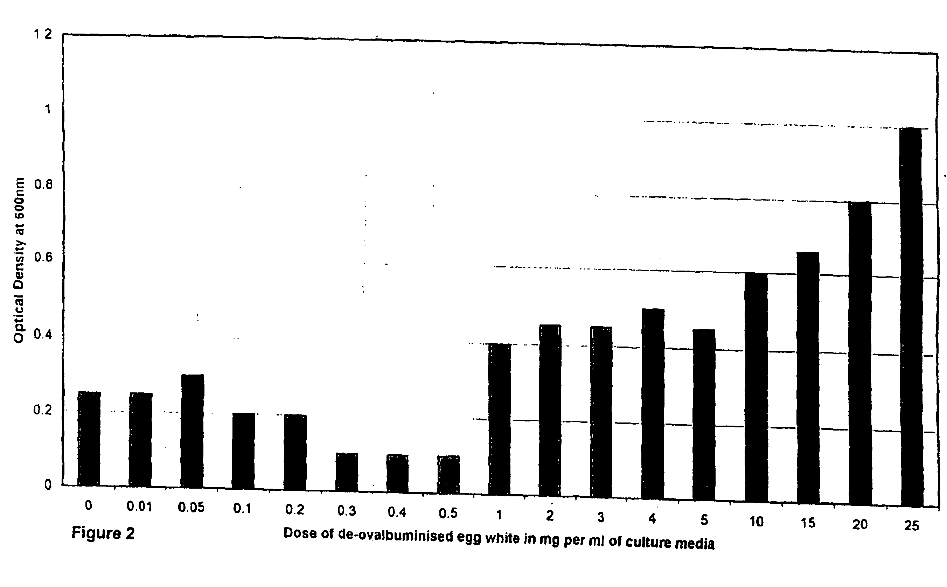

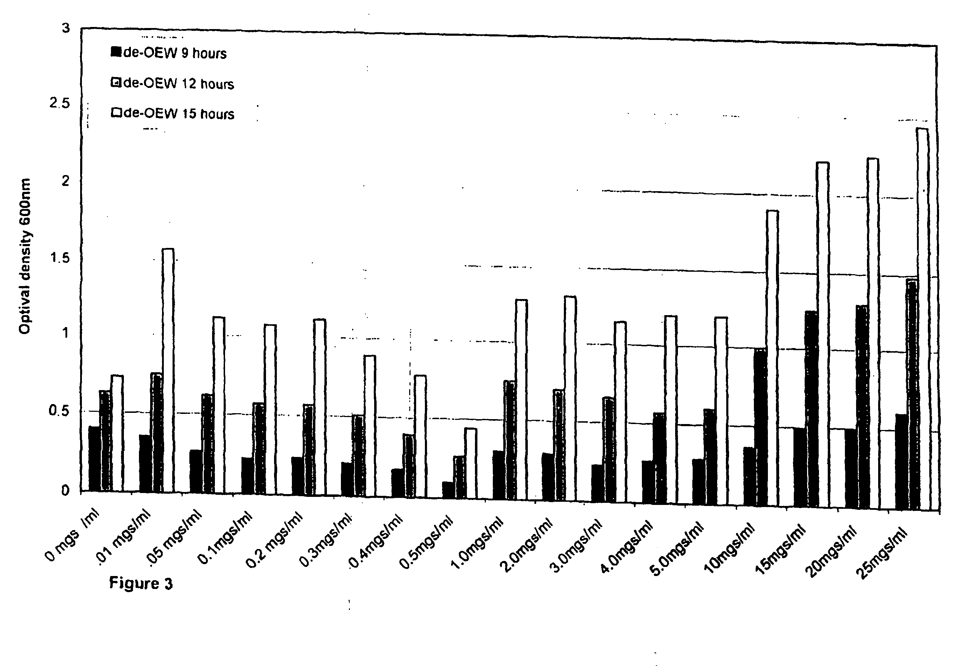

The effect of de-OEW on the growth of *St. mutans* was investigated and the results are shown in Figs. 2 and 3.

Fig. 2 illustrates the inhibitory effect of de-OEW on the growth of *Streptococcus mutans* in 80% SBNB at nine hours after inoculation. 80% SBNB is Nutrient Broth from Oxoid at 80% of normal strength supplemented with 2% sucrose and buffered at pH 6.8 with 0.5% potassium phosphate dibasic. The inhibitory effect is clearly demonstrated between 0.2 mg *per* ml and 1.0 mg *per* ml. As demonstrated in a separate experiment the inhibitory effect is continued throughout the growth cycle as shown in Fig. 3 with sample points at 9, 12 and 15 hours.

Unlike antibiotics or antiseptics the innate immune system does not have a bactericidal effect. The duality of inhibition and stimulation is a unique feature which mirrors the properties of the human mucous (saliva), where total antisepsis of the mouth is undesirable.
Some of the individual constituents of egg white have been more fully characterised than those of the egg yolk, (see Staedelman & Cotterill, Egg Science and Technology pages 119-137). The major components together with the properties conventionally assigned to them are summarised in Table 2.

**Table 2**

| Constituents of Egg White | | |
|---|---|---|
| Ovalbumin | 54% | phospoglycoprotein |
| Ovotransferrin | 12% | binds metallic ions |
| Ovomucoid | 11% | trypsin inhibitor |
| Ovomucin | 3.5% | viscous sialoprotein |
| Lysozyme | 3.4% | lytic activity against some |
| | | bacteria |
| G2 Globulin | 4% | unknown |
| G3 Globulin | 4% | unknown |
| Ovoinhibitor | 1.5% | inhibits serine protease |
| Ovoglycoprotein | 1 % | unknown |
| Ovoflavoprotein | 0.8% | binds riboflavin |
| Ovomacroglobulin | 0.5% | antigenic |
| Cystatin | 0.05% | inhibits thiol protease |
| Avidin | 0.05% | binds biotin |
| Total | 96% | |

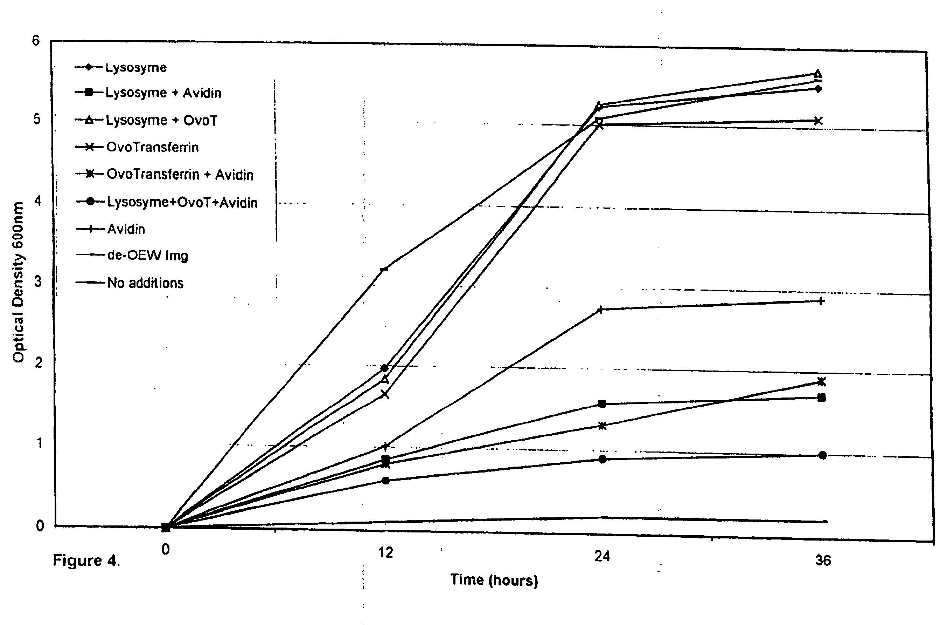

Three of the constituents are known to inhibit the growth of microorganisms these are lysozyme, ovotransferrin and avidin. The inhibitory properties of lysozyme are attributable to its enzymatic action against the cell wall of certain bacteria (not yeast).
Ovotransferrin is known to bind many metal ions including iron, so depleting its availability in the media. Avidin binds biotin and so denies its availability to those organisms that require this as an essential growth factor (including yeast). The relative potency of each of these ingredients on the growth of *Candida albicans* is illustrated in Fig. 4. Neither lysozyme nor ovotransferrin on its own inhibits growth, while avidin effects an approximate 50% reduction. All three in combination achieved an 80% reduction in growth, so demonstating synergistic activity between them. De-ovalbuminised egg white reduced growth by as much as 98%.

The relative percentage concentration of the known constituents of de-ovalbuminised egg white is shown in Table 3.

**Table 3**

| Approximate relative percentage concentration of the known constituents of de-ovalbuminised egg white | |
|---|---|
| Ovotransferrin | 24% |
| Ovomucoid | 24% |
| Ovomucin | 8% |
| Lysozyme | 8% |
| G2 Globulin | 8% |
| G3 Globulin | 8% |
| Ovoinhibitor | 4% |
| Ovoglycoprotein | 3% |
| Ovoflavoprotein | 2.5% |
| Ovomacroglobulin | 2% |
| Cystatin | 0.2% |
| Avidin | 0.2% |
| Total | 91.9% |

On the basis of Table 3 1.0 mg of de-ovalbuminised egg white will contain less than 0.25 mg of ovotransferrin, less than 0.1 mg of lysozyme and less than 0.05 mg of avidin.

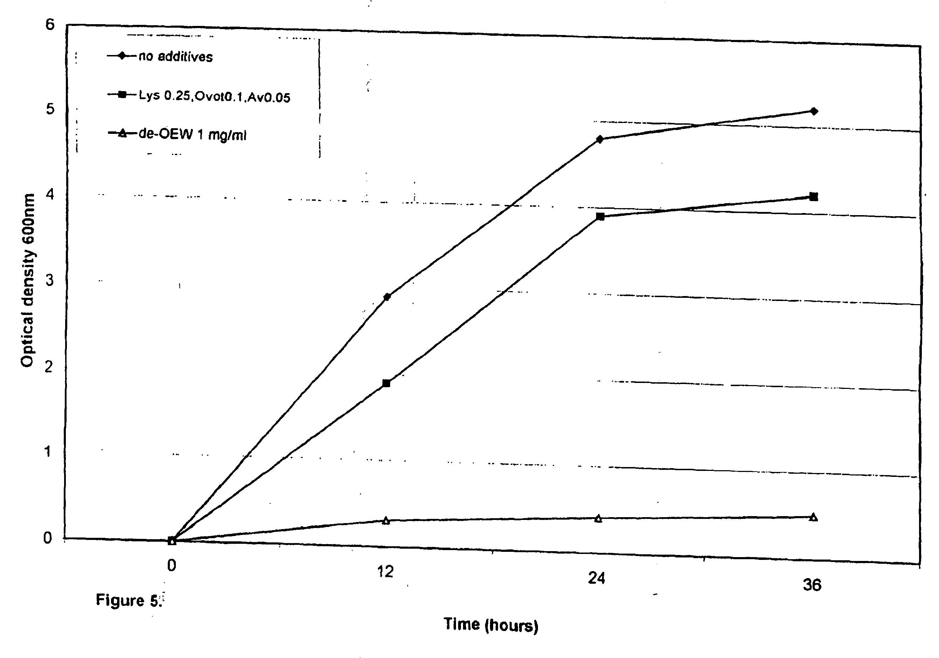

Fig. 5 illustrates the effect of a combination of ovotransferrin (0.25 mg/ml), lysozyme (0.1 mg/ml) and avidin (0.05 mg/ml) on the growth of *C. albicans* in comparison to the effect of 1.0 mg/ml of de-ovalbuminised egg white, illustrating that the greater inhibitory effect of de-ovalbuminised egg white is not due solely to these three ingredients.

### Example 8

### Formulation of synthetic saliva

De-ovalbuminised egg white (as prepared in Example 7) and enriched de-lipidised egg yolk (as described in Example 5) were combined in equal quantities by weight and dissolved in a solution of organic salts with the following final concentrations.

| Constituent | Concentration per litre |
|---|---|
| Innate immune constituents | 10 gram |
| Calcium | 2 mM |
| Magnesium | 0.2 mM |
| Sodium | 20 mM |
| Potassium | 20 mM |
| Ammonia | 5 mM |
| | |
| Hydrogen phosphate (divalent) | 20mM |
| Dihydrogen phosphate (monovalent) | 20mM |
| Bicarbonate | 20mM |

The pH of the solution was titrated to 6.7 using 0.1 Molar hydrochloric acid or 0.1 Molar ammonium hydroxide.

The formulation had a consistency similar to fresh whole saliva, with a comparable rheology and a resistance to drying equivalent to that of saliva.

### Example 9

### Inhibition of calcium precipitation

Calcium will precipitate out of a saturated solution of calcium chloride or calcium carbonate onto any suitable surface that provides a seed for initiation of crystal growth.

A solution of 4mM calcium chloride was prepared in 50mM HEPES buffer at pH 7.2. An equal weight of both de-ovalbuminised egg white (as prepared in Example 7) and de-lipidised enriched egg yolk (as prepared in Example 5) was added to this solution to give a final concentration of 0.01%w/v (0.1 mg/ml). 8 ml of this solution was added to 1.6 grams of BDH hydroxyapatite beads in a 25ml 'Sterilin' universal bottle. After equilibration at room temperature, 8.0 ml of 50mM HEPES pH 7.2 containing 15mM potassium dihydrogen phosphate was added. The effect of the potassium phosphate in this solution was to create a saturated solution with respect to the calcium ion.

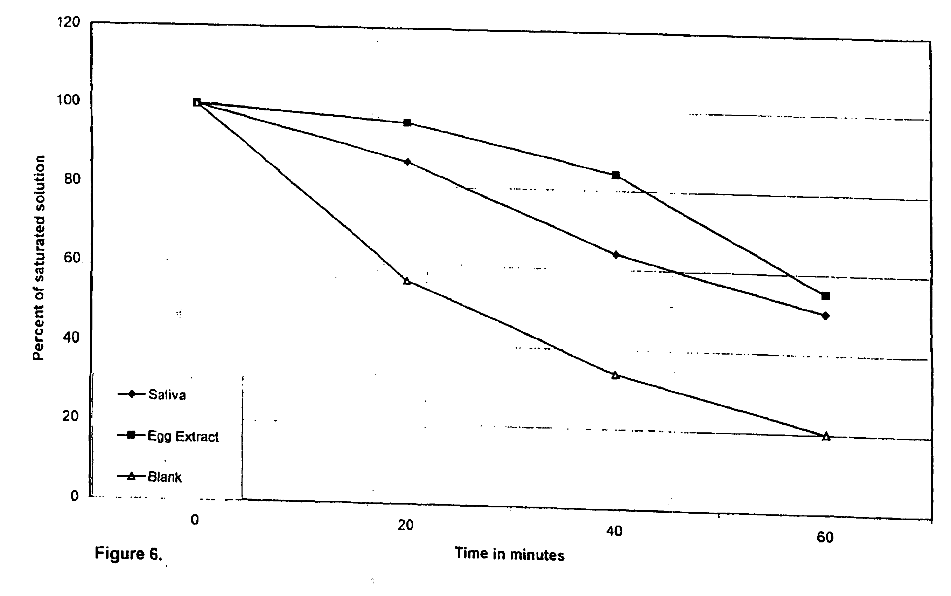

Loss of calcium from solution was measured using a Hanna Ion Specific Meter with Hanna reagents for the detection of calcium in accordance with the method described by Van der Reijden, W.A. et al; (Caries Research 1997;31;216-223). The percentage loss of calcium from solution was compared with a blank consisting of no added protein, a control with saliva saturated beads and the test solution consisting of the innate constituents of egg as described above. The results are presented in Fig. 6. After 40 min., there was a 65% reduction in the concentration of calcium in solution in the blank, in the presence of saliva the percentage reduction was 35%, while egg proteins gave superior maintenance of the solution allowing just 18% reduction.

### Example 10

### Incorporation of Polyols

Formulations of the innate immune constituents as described herein have the potential to replace fluoride as an active ingredient in toothpaste and other dentifrices and oral hygiene formulations providing broad spectrum antimicrobial benefits on par with fluoride in the case of dental caries and with much wider benefits in the control of gum disease, oral thrush and halitosis.

In this Example it is shown that the innate immune constituents of egg amplify the effect of xylitol and that xylitol extends the inhibitory concentration of the innate egg proteins. There is therefore a synergistic effect between the two, presenting an increased benefit from incorporation of low concentrations of the polyols in formulations of the innate immune constituents of egg.

*St. mutans* was cultured (test tube culture) in an 80% sucrose buffered nutrient broth (SBNB) medium. The medium is described as 80% SBNB because the final concentration of nutrient broth is 80% of normal.

The medium was prepared as follows:

| | |
|---|---|
| Oxoid nutrient broth | 20.8 grams |
| di-potassium hydrogen phosphate | 17.9 grams |
| sucrose | 40 grams |
| distilled water | 1 litre |

The pH of the above solution was adjusted to 6.8 with hydrochloric acid.

5.0ml aliquots of the above were dispensed into 25ml test tubes which were then sealed with cotton wool and autoclaved to sterilise them.

Solutions of the test material, being the innate immune system proteins of egg with or without polyols added at varying concentrations, were prepared in 0.1m phosphate buffer at pH 6.8 and sterilised by filtration using 0.2 micron capsule filters.

Volumes of the test material were added aseptically to the sterile media tubes in amounts which achieved specified concentrations of test material when the total volume of each tube was made up to 10ml with sterile 0.1 M phosphate buffer at pH 6.8.

The prepared tubes were innoculated with 0.1 ml of *St. mutans* culture grown for 12 hours in 80% SBNB without test material present. The inoculated tubes were incubated in a water bath at 37°C and the increase in Optical Density was measured using an EEL Colorimeter suitably filtered at 600nm. Each test series had a blank consisting of uninoculated media and a control consisting of inoculated media without test material.

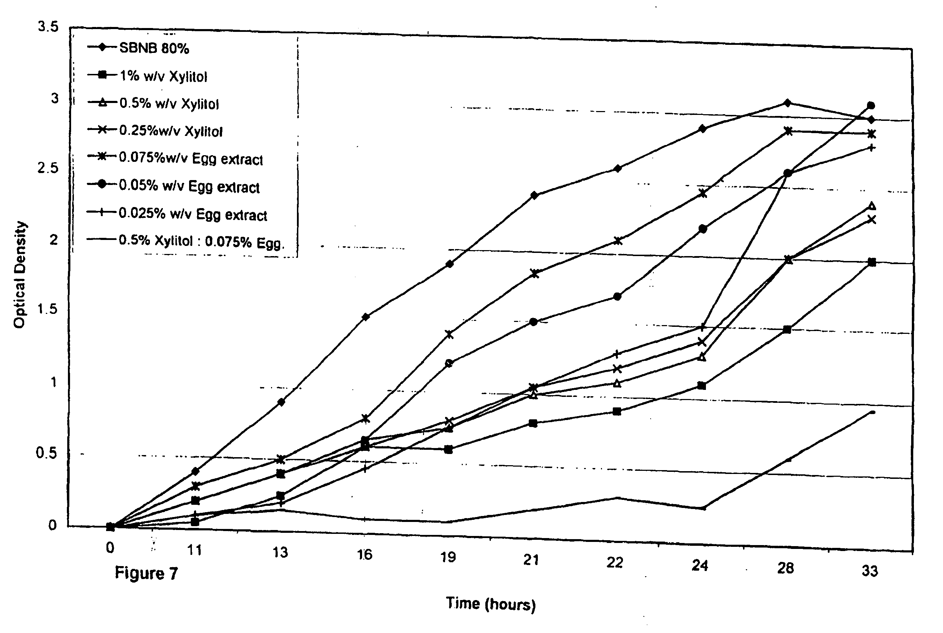

The results are depicted in Figs. 7-9.

Fig. 7 represents the effect of incorporating xylitol in 80% SBNB with *St. mutans.* There is increasing inhibition of growth from 0.25% w/v to 1.0% w/v xylitol. The same series shows the inhibitory effect of the innate immune system proteins of egg. ranging from 0.025%w/v up to 0.075%w/v. A combination of 0.5% xylitol with 0.075% innate immune system constituents of egg white extract as prepared in Example 7 provides amplified inhibition as shown. It should be noted that this series also demonstrates the inverse inhibitory effect of the innate immune system proteins described earlier.

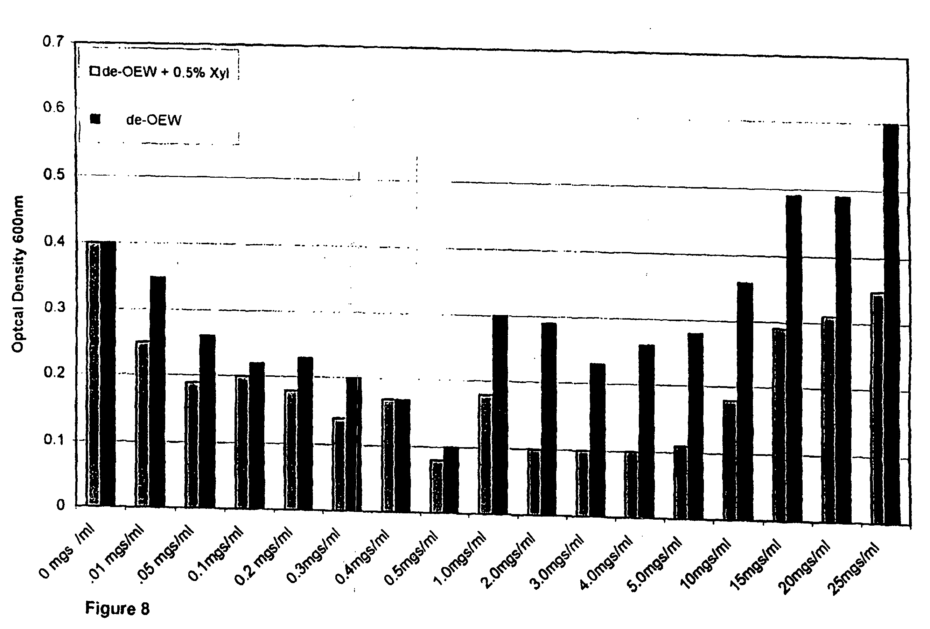

Fig. 8 presents the results of a nine hour growth of *St. mutans* in 80% SBNB with increasing concentration of the extract prepared in Example 7. The same series is presented adjacent supplemented with 0.5% xylitol. The xylitol has increased the inhibitory concentration 'window' from 0.2-1.0mg/ml with egg extract on its own to 0.2-5.0mg/ml with 0.5% xylitol added.

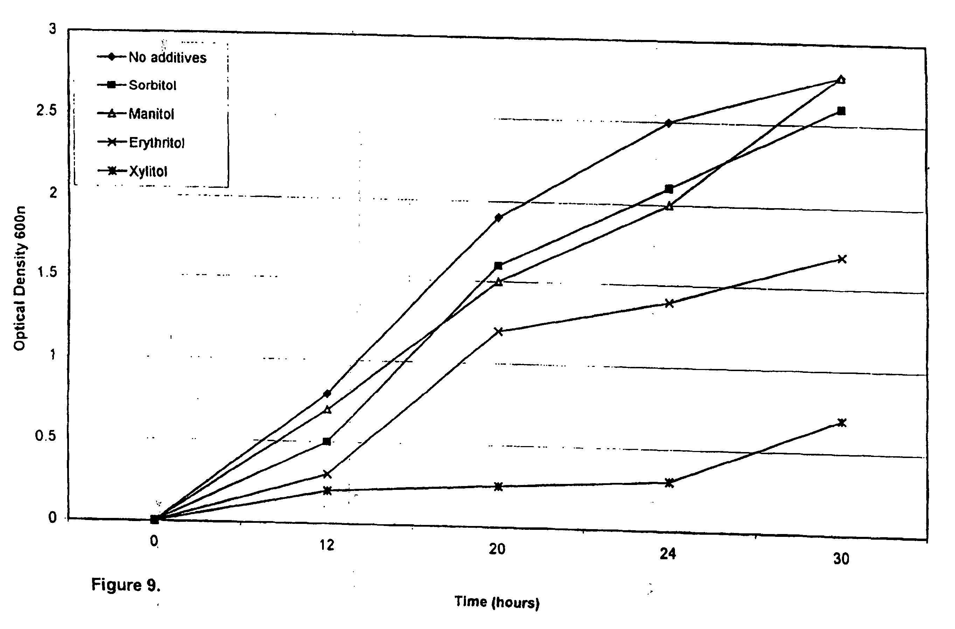

Fig. 9 illustrates the effect of mannitol, sorbitol, erythritol and xylitol at 0.5%w/v with 0.075% w/v of the extract prepared in Example 7 on the growth of *St. mutans* in 80% SBNB. Xylitol provides the optimal inhibition under the test conditions, other polyols may provide similar inhibition and have different inhibitory concentrations.

### Example 11

### Extraction and Enrichment of Innate Immune System Constituents from Milk

The protein content of milk is different to that of eggs and the dispersal of the innate system proteins in non-functional materials also differs, it is therefore necessary to modify the extraction procedure relative to that employed for egg to allow for these individual differences.

The lipid fraction of milk was removed by freezing at -70°C for twenty four hours followed by centrifugation on thawing and filtration of the supernatant. De-lipidisation is not necessary in the case of eggs but the dispersion of the innate system proteins in milk requires that the fat globules are disrupted and separated.

The pH of the resulting supernatant was reduced to 3.5 with citric acid and dialysed against de-ionised water to remove metallic ions and low molecular weight constituents which are bound to many of the functional groups of the innate system proteins of the milk. The de-ionised protein solution was concentrated by ultra-filtration using an exclusion membrane with a 'cut-off' of no more than 10,000. The protein solution was adjusted to 10 mg *per* ml using 10mM potassium phosphate buffer. Extraction of the innate system proteins was then carried out using baker's yeast as described in Example 5.

### Example 12

### Comparison of In Vitro Efficacy of enriched egg and milk extracts with hyperimmune antibody from egg, directed against Streptooccus mutans

The *in vitro* efficacy of the enriched innate system constituents of eggs with those from milk and with hyperimmune egg antibody directed against *St. mutans* was carried out. *St. mutans* will adhere strongly to saliva coated hydroxyapatite beads in the same way as this organism adheres to the surface of the dental enamel. It is necessary to first coat the hydroxyapatite beads with fresh whole saliva as the anchorage of *St. mutans* is to salivary proteins on the surface of the teeth.

0.1 gram aliquots of hydroxyapatite beads were weighed into 'Sterilin' (Sterilin is a Trade Mark) centrifuge tubes and incubated with 2.0 ml of fresh saliva for 2 hours with gentle agitation at 30°C. The hydrated beads were washed twice in distilled water to remove residual saliva.

*St. mutans* was cultured for twelve hours in Brain Heart Infusion Broth supplemented with 2% sucrose. The broth cultures were washed twice in phosphate buffered saline at pH 7.0 by centrifugation. The recovered cells were re-suspended in phosphate buffered saline and diluted to an Optical Density of 1.0 at 600nm.

Hyperimmune anti-*st. mutans* egg antibodies were prepared as described in Example 6. The antibody was diluted to 10mg/ml with 10mM phosphate buffer at pH 7. A solution consisting of equal weights of enriched egg yolk extract (prepared as described in Example 5) and de-ovalbuminised egg white (prepared as described in Example 7) was diluted to 10mg/ml with 10mM phosphate buffer. Enriched innate system proteins from milk, prepared as described in Example 11 were diluted to 10 mg/ml. 1.0 ml of each test solution was added to 1.0 ml of prepared *St. mutans* cells and incubated for 1 hour at 30°C. A blank determination was conducted using phosphate buffered saline instead of a test protein solution.

After incubation the test solutions were washed twice with phosphate buffered saline, re-suspended in 1.0 ml of the same buffer and added to the saliva coated hydroxyapatite beads. The beads together with the test solutions were incubated for 2 hours at 30°C, then washed carefully five times with sterile phosphate buffered saline to remove any bacterial cells not adhering to the beads.

An approximate estimate of the numbers of bacteria adhering to each bead was obtained by staining the beads with crystal violet and washing with alcohol followed by microscopic examination at x500. Standard Gram staining techniques are also suitable.

A more accurate measure of the relative numbers of bacteria adhering to each bead is obtained by the method reported by Hatta, H, *et. al*. ((1997) *supra*); an ultrasonic probe was used to detach the adhering bacteria.

In this Example the bacteria were detached using the same high ionic strength buffer as was used in Example 5 to desorb the enriched egg proteins from yeast. To each test sample 1.0 ml of 1 Molar phosphate buffer at pH 4 with 0.01% Tween was added. After vigorous agitation for 30 seconds the supernatant was immediately diluted in serial fashion. Using standard plate count techniques, 1 ml aliquots of each dilution were spread on the surface of fresh blood agar plates. After incubation the plates were inspected and the number of colonies counted; a multiplication factor based on the dilution was used to determine the number of viable cells in the original sample.

An average of three tests is presented in Table 4.

**Table 4**

| Relative inhibition of adhesion of *S. mutans* to hydroxyapatite beads | |
|---|---|
| | Numbers of bacteria adhering to the beads |
| Cell suspension | 60 x 10⁸ |
| Blank | 120 x 10⁶ |
| Milk extract | 175 x 10⁵ |
| Hyperimmune egg antibody | 105 x 10⁴ |
| Enriched egg extract | 80 x 10⁴ |

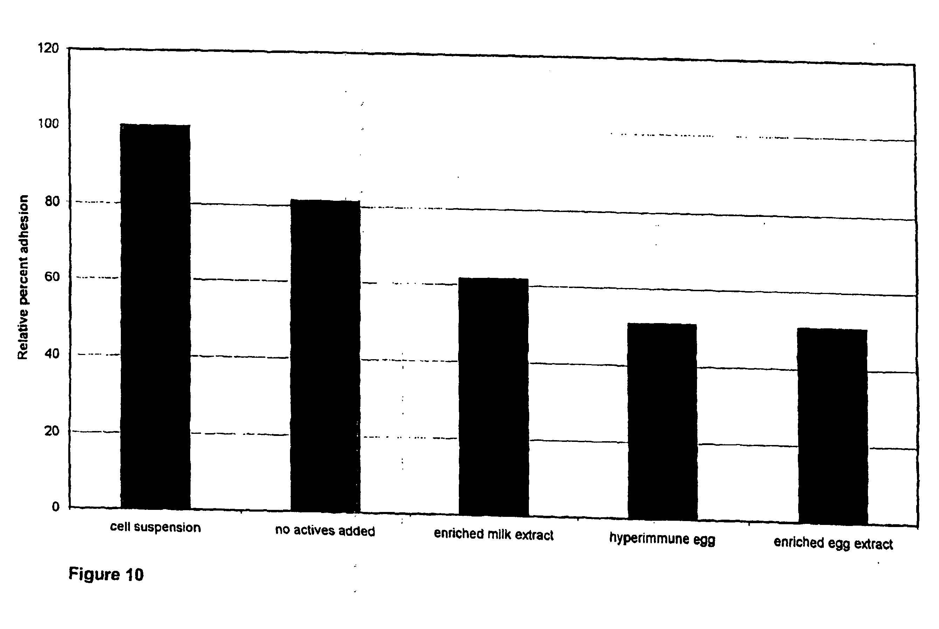

The results are also illustrated by Fig. 10.

## Claims

1. Use of those innate immune constituents of a maternal immune secretion selected from egg fluid and milk, which adhere to the surface of a microbial cell, said innate immune constituents being extracted from the maternal immune secretion, said extract having anti-microbial activity characteristic of a human mucosal secretion selected from saliva and genital secretion and which is tolerated by humans, in the manufacture of a medicament for the treatment and/or prophylaxis of a condition of the human body **characterised by** a loss of said mucosal secretion or the normal biological properties thereof or which is responsive to said mucosal secretion, the conditions of the human body being selected from a condition of the buccal cavity or vaginal infection, including yeast vaginitis.

2. Use according to Claim 1, wherein the egg fluid is selected from (i) egg fluid obtained from a domestic hen; (ii) egg fluid derived from egg yolk; (iii) egg fluid derived from delipidised egg yolk; (iv) egg fluid derived from egg white; (v) egg fluid derived from de-ovalbuminised egg white; and (vi) a mixture of delipidised yolk and de-ovalbuminised egg white.

3. Use according to any preceding claim, wherein the medicament is a replica of said human mucosal secretion.

4. Use according to any preceding claim, wherein said medicament comprises a maternal immune secretion enriched with said extract.

5. Use according to any preceding claim, wherein said innate immune constituents of the maternal immune secretion adhere to the surface of *Saccharomyces cerevisiae*.

6. Use according to any preceding claim, wherein the medicament is used in the treatment of xerostomia.

7. Use according to any one of Claims 1-5, wherein the medicament is used in the treatment and/or prophylaxis of oral infection.

8. Use according to any preceding claim, wherein said condition of the buccal cavity is selected from dental caries; a condition of the buccal cavity **characterised by** inflammation of the gums; and halitosis.

9. Use according to any one of Claims 6-8, wherein the medicament is used in the form of a mouthwash.

10. Use according to any one of Claims 5, 7-9, wherein the medicament is used in the general prophylaxis of dental caries or gum disease in individuals with no pre-disposition to other oral disease.

11. Use according to any preceding claim, wherein the medicament comprises one or more inorganic salts characteristic of said human mucosal secretion.

12. Use according to any preceding claim wherein the medicament is administered simultaneously, separately or sequentially with a plant extract which potentiates the effect of said extract.

13. Use according to Claim 12, wherein the plant extract is obtained from Vaccinium myrtilis, Zea mais, Melissa officianalis or Pilocarpus microphilis.

14. A formulation for use in the treatment and/or prophylaxis of a condition of the buccal cavity **characterised by** a loss of normal saliva or the normal biological properties thereof or which is responsive to normal saliva, which comprises an extract as defined in Claim 1 and a polyol selected from erythritol, mannitol, sorbitol and xylitol.

15. A formulation according to Claim 13 or 14, which is in the form of a chewing gum or a toothpaste.

## Patentansprüche

1. Verwendung der angeborenen Immunbestandteile des maternalen Immunsekrets ausgewählt aus Eiflüssigkeit und Milch, die an der Oberfläche einer Mikrobenzelle haften, wobei die angeborenen Immunbestandteile aus dem maternalen Sekret extrahiert sind, und der Extrakt antimikrobielle Aktivität aufweist, die für ein humanes Schleimhautsekret charakteristisch ist, ausgewählt aus Speichel und Genitalsekret und von Menschen toleriert wird, für die Herstellung eines Medikaments zur Behandlung und/oder Prophylaxe einer Erkrankung des humanen Körpers, die **gekennzeichnet ist durch** einen Verlust des Schleimhautsekrets oder der normalen biologischen Eigenschaften davon oder die auf das Schleimhautsekret anspricht, wobei die Erkrankung des humanen Körpers ausgewählt ist aus einer Erkrankung der Mundhöhle oder Vaginalinfektion, einschließlich Hefe-Vaginitis.

2. Verwendung nach Anspruch 1, wobei die Eiflüssigkeit ausgewählt ist aus: (i) von einem Haushuhn gewonnener Eiflüssigkeit; (ii) sich von Eigelb herleitender Eiflüssigkeit; (iii) sich von delipisiertem Eigelb herleitender Einflüssigkeit; (iv) sich von Eiweiß herleitender Eiflüssigkeit; (v) sich von deovalbumisiertem Eiweiß herleitender Eiflüssigkeit; und (vi) einem Gemisch aus delipisiertem Eigelb und deovalbumisiertem Eiweiß.

3. Verwendung nach einem der Ansprüche 1 - 3, wobei das Medikament eine Nachbildung des humanen Schleimhautsekrets ist.

4. Verwendung nach einem der vorhergehenden Ansprüche, wobei das Medikament ein mit dem Extrakt angereichertes maternales Immunsekret umfasst.

5. Verwendung nach einem der vorhergehenden Ansprüche, wobei die angeborenen Immunbestandteile des maternalen Immunsekrets an die Oberfläche von *Saccharomyces cerevisiae* haften.

6. Verwendung nach einem der vorhergehenden Ansprüche, wobei das Medikament bei der Behandlung von Xerostomie verwendet wird.

7. Verwendung nach einem der Ansprüche 1-5, wobei das Medikament bei der Behandlung und/oder Prophylaxe einer Mundinfektion verwendet wird.

8. Verwendung nach einem der vorhergehenden Ansprüche, wobei die Erkrankung der Mundhöhle ausgewählt ist aus Zahnkaries, einer durch Entzündung des Zahnfleischs gekennzeichneten Erkrankung der Mundhöhle und Halitosis.

9. Verwendung nach einem der Ansprüche 6-8, wobei das Medikament in der Form eines Mundwassers verwendet wird.

10. Verwendung nach einem der Ansprüche 5, 7 - 9, wobei das Medikament bei der allgemeinen Prophylaxe von Zahnkaries oder einer Zahnfleischerkrankung bei Menschen verwendet wird, die keine Prädisposition für eine andere Mundkrankheit haben.

11. Verwendung nach einem der vorhergehenden Ansprüche, wobei das Medikament ein oder mehrere anorganische(s) Salz(e) umfasst, das/die für das humane Schleimhautsekret charakteristisch ist/sind.

12. Verwendung nach einem der vorhergehenden Ansprüche, wobei das Medikament gleichzeitig, getrennt oder sequenziell mit einem die Wirkung des Extrakts potenzierenden Pflanzenextrakt verabreicht wird.

13. Verwendung nach Anspruch 12, wobei der Pflanzenextrakt aus Vaccinium myrtilis, Zea mays, Melissa officinalis oder Pilocarpus microphilis gewonnen wird.

14. Formulierung zur Verwendung bei der Behandlung und/oder Prophylaxe einer Erkrankung der Mundhöhle, **gekennzeichnet durch** einen Verlust von normalem Speichel oder der normalen Eigenschaften davon oder die auf normalen Speichel anspricht, die einen nach Anspruch 1 definierten Extrakt und ein Polyol umfasst, ausgewählt aus Erythritol, Mannitol, Sorbitol und Xylitol.

15. Formulierung nach Anspruch 13 oder 14, die in der Form eines Kaugummis oder einer Zahnpaste vorliegt.

## Revendications

1. Utilisation des constituants immuns innés d'une sécrétion immune maternelle choisie parmi le liquide d'oeuf et le lait, qui adhèrent à la surface d'une cellule microbienne, lesdits constituants immuns innés étant extraits de la sécrétion immune maternelle, ledit extrait ayant une activité antimicrobienne caractéristique d'une sécrétion muqueuse humaine choisie parmi la salive et les sécrétions génitales et qui est toléré par l'homme, dans la fabrication d'un médicament pour le traitement et/ou la prophylaxie d'une affection du corps humain, **caractérisée par** une perte de ladite sécrétion muqueuse ou des propriétés biologiques normales de celle-ci ou qui est sensible à ladite sécrétion muqueuse, les affections du corps humain étant choisies parmi une affection de la cavité buccale ou une infection vaginale, incluant la vaginite à levure.

2. Utilisation selon la revendication 1, dans laquelle le liquide d'oeuf est choisi parmi (i) le liquide d'oeuf obtenu à partir d'une poule domestique ; (ii) le liquide d'oeuf dérivé du jaune d'oeuf ; (iii) le liquide d'oeuf dérivé du jaune d'oeuf délipidé ; (iv) le liquide d'oeuf dérivé du blanc d'oeuf ; (v) le liquide d'oeuf dérivé du blanc d'oeuf débarrassé de l'ovalbumine ; et (vi) un mélange de jaune d'oeuf délipidé et de blanc d'oeuf débarrassé de l'ovalbumine.

3. Utilisation selon l'une quelconque des revendications précédentes, dans laquelle le médicament est une réplique de ladite sécrétion muqueuse humaine.

4. Utilisation selon l'une quelconque des revendications précédentes, dans laquelle ledit médicament comprend une sécrétion immune maternelle enrichie avec ledit extrait.

5. Utilisation selon l'une quelconque des revendications précédentes, dans laquelle lesdits constituants immuns innés de la sécrétion immune maternelle adhèrent à la surface de *Saccharomyces cerevisiae.*

6. Utilisation selon l'une quelconque des revendications précédentes, dans laquelle le médicament est utilisé dans le traitement de la xérostomie.

7. Utilisation selon l'une quelconque des revendications 1 à 5, dans laquelle le médicament est utilisé dans le traitement et/ou la prophylaxie d'infections orales.

8. Utilisation selon l'une quelconque des revendications précédentes, dans laquelle ladite affection de la cavité buccale est choisie parmi les caries dentaires ; une affection de la cavité buccale **caractérisée par** une inflammation des gencives ; et la mauvaise haleine.

9. Utilisation selon l'une quelconque des revendications 6-8, dans laquelle le médicament est utilisé sous la forme d'un bain de bouche.

10. Utilisation selon l'une quelconque des revendications 5, 7-9, dans laquelle le médicament est utilisé dans la prophylaxie générale des caries dentaires ou de la maladie des gencives chez des personnes n'ayant aucune prédisposition pour d'autres maladies orales.

11. Utilisation selon l'une quelconque des revendications précédentes, dans laquelle le médicament comprend un ou plusieurs sels minéraux caractéristiques de ladite sécrétion muqueuse humaine.

12. Utilisation selon l'une quelconque des revendications précédentes, dans laquelle le médicament est administré de manière simultanée, séparée ou séquentielle avec un extrait de plante ce qui potentialise l'effet dudit extrait.

13. Utilisation selon la revendication 12, dans laquelle l'extrait de plante est obtenu à partir de Vaccinium myrtilis, Zea mays, Melissa officinalis ou Pilocarpus microphylis.

14. Formulation pour une utilisation dans le traitement et/ou la prophylaxie d'une affection de la cavité buccale **caractérisée par** une perte de la salive normale ou des propriétés biologiques normales de celle-ci, ou qui est sensible à la salive normale, comprenant un extrait tel que défini selon la revendication 1 et un polyol choisi parmi l'érythritol, le manitol, le sorbitol et le xylitol.

15. Formulation selon la revendication 13 ou 14, qui est sous la forme d'une gomme à mâcher ou d'une pâte dentifrice.
